# EUROPEAN PATENT SPECIFICATION

(11) **EP 1 838 166 B1**
(45) Date of publication and mention of the grant of the patent: **08.08.2012**
(21) Application number: 05855800.8
(22) Date of filing: 29.12.2005
(51) Int. Cl.: A23K 1/17, A23K 1/18

(54) **COMPOSITIONS AND METHODS FOR IMPROVING KIDNEY FUNCTION**
ZUSAMMENSETZUNGEN UND VERFAHREN ZUR VERBESSERUNG DER NIERENFUNKTION
COMPOSITIONS ET PROCEDES POUR AMELIORER LA FONCTION RENALE

(30) Priority: 29.12.2004 US 24541
(43) Date of publication of application: 03.10.2007
(73) Proprietor: Hill's Pet Nutrition, Inc., Topeka, KS 66603 (US)
(72) Inventor: JEWELL, Dennis, Edward, Lawrence, Kansas 66049 (US)
(74) Representative: Daniels, Jeffrey Nicholas
(86) International application number: PCT/US2005/047301
(87) International publication number: WO 2006/071952

(56) References cited:
- US-A- 6 039 952
- US-A1- 2001 043 983
- US-A1- 2004 105 879
- GRECO D S: "DIETARY CONSIDERATIONS FOR DOGS WITH CHRONIC RENAL FAILURE" COMPANION ANIMAL PRACTICE, vol. 1, no. 1, 1987, pages 54-56, 62, XP009095388 ISSN: 0894-9794
- ANONYMOUS: "Ami Products - Ingredients" INTERNET CITATION, [Online] 24 June 2004 (2004-06-24), XP002446899 Retrieved from the Internet: URL:http://ami.aminews.net/en_ingredienti. html> [retrieved on 2007-08-14]
- POLZIN D J ET AL: "Dietary management of feline chronic renal failure: where are we now? In what direction are we headed?" JOURNAL OF FELINE MEDICINE AND SURGERY, WB SAUNDERS, LONDON, GB, June 2000 (2000-06), pages 75-82, XP002446902 ISSN: 1098-612X

## Description

### BACKGROUND OF THE INVENTION

### Field of the Invention

This invention relates generally to compositions and methods for improving kidney function and particularly to the use of food compositions for improving kidney function.

### Description of the Related Art

It has been postulated since 1956 that the production of active oxygen species or free radicals during aerobic respiration results in oxidative damage that hastens aging and death in animals (Beckman, K, et al., "The Free Radical Theory of Aging natures," Phys. Rev., 78: 547-581 (1998)). Active oxygen species cause aging through various mechanisms, including directly damaging cellular DNA (Cutler, R., "Antioxidants and aging", Am. J. Clin. Nutr., 53: 373S-379S (1991) and lipids and proteins (Tylicki, L., et al. "Antioxidants: A Possible Role in Kidney Protection," Kid. B1. Press. Res., 26: 303-314 (2003)). Free radicals, often produced in the mitochondria, where aerobic respiration occurs; damage mitochondrial DNA, proteins, and lipids, e.g., U.S. Patent App. Pub. No. US 2003/0060503.

It has also been postulated that active oxygen species may play a role in causing kidney disease (Ongajooth L., et al. "Role of Lipid Peroxidation, Trace Elements and Antioxidant Enzymes in Chronic Renal Disease Patients," J. Med. Assc. Thai., 79:791-800 (1996)). Several mechanisms have been proposed to account for this increase in renal failure, e.g., Hasselwander, et al. "Oxidative Stress in Chronic Renal Failure," Free Rad. Res. 29:1-11 (1998); Shah, S., "The Role of Reactive Oxygen Metabolites in Glomerular Disease," Annu. Rev. Physiol., 57:245-62 (1995)), but scientific studies to date are inconclusive regarding whether antioxidant treatment is beneficial to those with kidney disease. Some studies indicate that there is a role for various antioxidant supplementations in the protection against kidney disease, e.g., Kedziora-Komatowska et al, "Effect of Vitamin E and Vitamin C Supplementation on Antioxidative State and Renal Glomerular Basement Membrane Thickness in Diabetic Kidney", Nephron Exp. Nephrol., 95:e134-e143 (2003). Other studies note the potential pro-oxidant properties of antioxidant supplements, concluding that there is not yet enough experimental evidence to recommend antioxidant supplements to alleviate kidney disease, e.g., Tylicki, L., et al.

In addition to an improvement of kidney function caused by supplementation of an animal's diet with antioxidants, kidney function may also benefit from the avoidance of mineral excess. Byproducts of protein digestion and phosphorus are among the primary toxins that must be removed from the blood by the kidneys. Thus, by decreasing protein and/or phosphorus consumption in an animal's diet, less stress is placed upon kidney function and therefore improves kidney health. In short, lowering the dietary intake of protein and/or phosphorus is beneficial to improving kidney function and/or lessening the progression of renal disease. See, e.g. U.S. Patent No. 6,306,442.

However, despite years of studies and developments relating to kidney disease and renal function, kidney disease and poor kidney function remain a major health problem. There is, therefore, a need for new methods and compositions for improving kidney function.

### SUMMARY OF THE INVENTION

It is, therefore, an object of the present invention to provide compositions and methods for improving kidney function.

It is another object of the invention to provide articles of manufacture in the form of kits that contain combinations of compositions and devices useful for improving kidney function.

These and other objects are achieved using a novel compositions for improving kidney function. The compositions comprise antioxidants and a reduced amount of protein and/or phosphorus as compared to the maximum amount of protein and phosphorus typically recommended for a healthy animal of the same species or breed. Generally, the compositions contain from about 25 to about 2,000 mg/kg vitamin C, from about 300 to about 2,000 IU/kg vitamin E, less than about 23% protein, and/or less than about 0.75% phosphorus. The description also provides methods comprising administering such compositions to animals susceptible to or suffering from kidney disease or administering the compositions to animals experiencing a decline in kidney function, particularly a decline due to aging. Kits comprising the composition components and optional renal drugs are provided.

Other and further objects, features, and advantages of the present invention will be readily apparent to those skilled in the art.

### DETAILED DESCRIPTION OF THE INVENTION

### Definitions

The term "animal" means any animal susceptible to or suffering from poor or impaired kidney function and therefore in need of improved kidney function. The methods and compositions of the invention are useful for a variety of human and non-human animals, including avian, bovine, canine, equine, feline, hicrine, murine, ovine, and porcine animals, and are particularly useful for companion animals such as canines and felines, including dogs and cats.

The term "renal drug" means any compound, composition, or drug useful for preventing or treating kidney disease or improving kidney function.

The term "in conjunction" means that one or more of the compositions and compounds (e.g., renal drugs or composition components) of the present invention are administered to an animal (1) together in a food composition or (2) separately at the same or different frequency using the same or different administration routes at about the same time or periodically. "Periodically" means that the compositions, food compositions, and compounds are administered on a dosage schedule acceptable for a specific composition, food composition, and compound and that the food compositions are administered or fed to an animal routinely as appropriate for the particular animal. "About the same time" generally means that the compositions, composition components, renal drugs, and food compositions are administered at the same time or within about 72 hours of each other. In conjunction specifically includes administration schemes wherein renal drugs are administered for a prescribed period and the compositions are administered indefinitely.

The term "antioxidant" means a substance capable of reacting with and neutralizing free radicals. Examples of such substances include beta-carotene, selenium, coenzyme Q10 (ubiquinone), lutein, tocotrienols, soy isoflavones, S-adenosylmethionine, glutathione, N-acetylcysteine, vitamin E, vitamin C, (-lipoic acid, and L-carnitine. Examples of foods containing useful levels of one or more antioxidants include but are not limited to ginkgo biloba, green tea, broccoli, citrus pulp, grape pomace, tomato pomace, carrot, spinach, and a wide variety of fruit meals and vegetable meals.

The term "single package" means that the components of a kit are physically associated in or with one or more containers and considered a unit for manufacture, distribution sale, or use. Containers include, but are not limited to, bags, boxes, bottles, shrink wrap packages, stapled or otherwise affixed components, or combinations thereof. A single package may be containers of individual antioxidants and/or food ingredients physically associated such that they are considered a unit for manufacture, distribution, sale, or use.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

All percentages expressed herein are by weight of the composition on dry matter basis unless specifically stated otherwise.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

### The Invention

In one aspect, the invention provides compositions for improving kidney function. The compositions comprise both a kidney function improving amount of one or more antioxidants and a reduced amount of protein and/or phosphorus as compared to the maximum amount of protein and phosphorus typically recommended for a healthy animal of the same species or breed. The invention is based upon the discovery that a reduction in certain nutrients in an animal's diet in conjunction with the dietary supplementation of certain antioxidants improves kidney function. Without being bound by theory, it is believed that the improvement results from decreased toxins in the blood due to the reduction of nutrients and decreased presence of oxygen species in the blood due to the presence of antioxidants.

Enhancing or improving kidney function means increasing the kidney's ability to remove waste or toxins from an animal's blood as compared to an earlier time. Generally, as animals age, there is a decrease in the total glomerular filtration caused by a declining ability of the kidneys to adequately filter urine. It is believed that this decrease in kidney function is caused by a decrease in nephron number and function.

Kidney function can be determined by methods known to skilled artisans. Generally, any of several blood indices may be used to determine kidney function, particularly the severity of decrease in kidney function or renal disease. Among these indices is serum urea nitrogen (SUN). SUN levels in the blood of an animal increase when the animal suffers from renal failure because damage to the kidney lessens the kidney's ability to adequately filter urea, a waste product. Kidney function can also be measured via the monitoring of glomerular filtration rate (GFR). The greater an animal's GFR, the better the kidneys are at removing waste and, therefore, functioning. GFR can be measured in a number of ways, including the use of an iohexol clearance test. Per this test, the intended animal first fasts for twelve or more hours. Then, iohexol, a radiographic contrast agent, is injected into the blood via an intravenous catheter. At 2, 3, and 4 hours after administration of iohexol, a minimum of 4 ml of blood (at least 1.2 ml serum) is obtained and tested. The rate at which iohexol in the blood is decreasing indicates the level at which the kidney is functioning. The overall functioning of the animal's kidneys can be measured via a comparison of that animal's GFR at one point in time versus the animal's GFR at a later point in time.

Any antioxidant that can provide improved kidney function can be used in the compositions and methods of the present invention. Suitable antioxidants include, but are not limited to, vitamin E, L-carnitine, a-lipoic acid, and vitamin C. Vitamin E can be in any form suitable for consumption by an animal including, but not limited to, any tocopherol or tocotrienol compound, any enantiomer or racemate thereof, and any mixture of such compounds having vitamin E activity. Vitamin E can be administered as any one or a mixture of different forms or in the form of various derivatives thereof such as esters, including vitamin E acetate, succinate, palmitate and the like, that exhibit vitamin E activity after ingestion by an animal. L-camitine can be administered as such or in the form of any of various derivatives of carnitine, such as salts, e.g., hydrochloride, fumarate and succinate salts, acetylated carnitine, and the like. a-Lipoic acid can be administered as such, as a lipoate derivative, for example as described in U.S. Patent No. 5,621,117, or as a racemic mixture, salt, ester or amide thereof. In one embodiment DL-a-lipoic acid is used. Vitamin C can be administered as ascorbic acid, for example L-ascorbic acid, or as various derivatives thereof such as calcium phosphate salt, cholesteryl salt, and ascorbate-2-monophosphate. Salts of vitamin C include, for example, sodium salt, calcium salt, zinc salt and ferrous salt. Esters include, for example, stearate, palmitate and like derivatives. Vitamin C or a derivative thereof can be in any physical form, for example, a liquid, a semisolid, a solid, or a heat stable form that exhibits vitamin C activity after ingestion by the animal. In various embodiments, the compositions comprise various combinations of vitamin E, vitamin C, L-carnitine, and a-lipoic acid.

The compositions of the present invention contain a kidney function improving amount of one or more antioxidants. The amount of antioxidants suitable for improving kidney function for a particular antioxidant is easily determined by the skilled artisan based upon the characteristics of the antioxidant(s). Generally, the composition comprises at least about 10 mg/kg of one or more antioxidants or its equivalent in other units, e.g., IU/kg for vitamin E. For example, compositions of the invention may contain vitamin C and/or vitamin E. In general, the concentration of vitamin C, if present in the compositions of the invention, is at least about 25 mg/kg (or from about 25 to about 2,000 mg/kg, or about 40 to about 1500 mg/kg, or about 50 to about 1000 mg/kg, or about 75 to about 500 mg/kg, or about 100 to about 200 mg/kg) based on the dry weight of the composition. In general, the concentration of vitamin E, if present in the composition of the invention, is at least about 300 IU/kg (or from about 400 to about 1700 IU/kg, or about 500 to about 1400 IU/kg, or about 600 to about 1100 IU/kg, or about 700-800 IU/kg) based on the dry weight of the composition. The weight ratio of vitamin C to vitamin E (as DL-alpha-tocopheryl acetate equivalents) is about 0.2:1 to about 7:1.

To reduce the stress on the kidney of an animal with reduced kidney function, the compositions of the invention also contain a reduced amount of protein and/or phosphorus as compared to the maximum typically recommended for a healthy animal of the same species or breed. For example, it has been recommended that the maximum be 23% of a typical dog's diet comprises protein. *See, eg.,* Small Animal Clinical Nutrition, 4th ed., p. 223 (2000). In general, the concentration of protein in the compositions of the invention is less than about 23% or less than about 20%, based on the dry weight of the composition. Relatedly, it has been recommended that at least 0.75% of a typical dog's diet comprise phosphorus. *See, e.g.,* Small Animal Clinical Nutrition, 4th ed., p. 223 (2000). In general, the concentration of phosphorus in the dog food compositions of the invention is less than about 0.75%, based on the dry weight of the composition. The concentration of phosphorus in the compositions of the invention may also be less than about 0.60%, based on the dry weight of the composition. The concentrations of phosphorus in the compositions of the invention may also be less than about 0.50%, based on the dry weight of the composition. The weight ratio of protein to phosphorus is about 17: 1 to about 110:1. The weight ratio of protein to antioxidants is about 410:1 to about 70:1.

The antioxidants are present at concentrations that are not deleterious to the intended animal's health. Thus, for example, the antioxidants are present at concentrations that do not cause undesirable toxic effects. Also, the protein and phosphorus are present in the compositions of the invention at concentrations that are sufficient to provide the intended animal sufficient dietary protein and phosphorus to maintain the overall health of the animal.

In one aspect, the invention provides a food composition for improving kidney function. The food ingredients useful in the present invention include any food ingredient suitable for consumption by an animal. Typical food ingredients include but are not limited to fats, carbohydrates, proteins, fibers, nutritional balancing agents such as vitamins, minerals, and trace elements, and mixtures thereof. Skilled artisans can select the amount and type of food ingredients for a typical food based upon the dietary requirements of the animal, e.g., the animal's species, age, size, weight, health, and function.

The food ingredient part of the food composition can comprise 100% of any particular food ingredient of can comprise a mixture of food ingredients in various proportions. In preferred embodiments, the food composition comprises a combination of food ingredients in amounts from about 0% to about 50% fat, from about 0% to about 75% carbohydrate, from about 0% to about 95% protein, from about 0% to about 40% dietary fiber, and from about 0% to about 15% of one or more nutritional balancing agents.

The fat and carbohydrate food ingredient is obtained from a variety of sources such as animal fat, fish oil, vegetable oil, meat, meat by-products, grains, other animal or plant sources, and mixtures thereof. Grains include wheat, corn, barley, and rice.

The protein food ingredient is obtained from a variety sources such as plants, animals, or both. Animal protein includes meat, meat by-products, dairy, and eggs. Meats include the flesh from poultry, fish, and animals such as cattle, swine, sheep, goats, and the like. eat by-products include lungs, kidneys, brain, livers, stomachs, and intestines. The protein food ingredient may also be free amino acids and/or peptides. Preferably, the protein food ingredient comprises meat, a meat by-product, dairy products, or eggs.

The fiber food ingredient is obtained from a variety of sources such as vegetable fiber sources, e.g., cellulose, beet pulp, peanut hulls, and soy fiber.

The nutritional balancing agents are obtained from a variety of sources known to skilled artisans, e.g., vitamin and mineral supplements and food ingredients. Vitamins and minerals can be included in amounts required to avoid deficiency and maintain health. These amounts are readily available in the art. The National Research Council (NRC) provides recommended amounts of such nutrients for farm animal See, e.g., Nutrient Requirements of Swine (10th Rev. Ed., Nat'l Academy Press, Wash. D.C., 1998), Nutrient Requirements of Poultry (9th Rev. Ed., Nat'l Academy Press, Wash. D.C., 1994), Nutrient Requirements of Horses (5th Rev. Ed., Nat'l Academy Press, Wash. D.C., 1989). The American Feed Control Officials (AAFCO) provides recommended amounts of such nutrients for dogs and cats. See American Feed Control Officials, Inc., Official publication, pp. 129-137 (2004). Vitamins generally useful as food additives include vitamin A, vitamin B1, vitamin B2, vitamin B6, vitamin B12, vitamin D, biotin, vitamin K, folic acid, inositol, niacin, and pantothenic acid. Minerals and trace elements useful as food additives include calcium, phosphorus, sodium, potassium, magnesium, copper, zinc, chloride, iron, selenium, iodine, and iron.

The compositions and food compositions may contain additions ingredients such as vitamins, minerals, fillers, palatability enhancers, binding agents; flavors, stabilizers, emulsifiers, sweeteners, colorants, buffers, salts, coatings, and the like known to skilled artisans. Stabilizers include substances that tend to increase the shelf life of the composition such as preservatives, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches. Specific amounts for each composition component, food ingredient, and other ingredients will depend on a variety of factors such as the particular components and ingredients included in the composition; the species of animal; the animal's age, body weight, general health, sex, and diet; the animal's consumption rate; the type of kidney disease being treated. Therefore, the component and ingredient amounts may vary widely and may deviate from the preferred proportions described herein.

In one embodiment, the composition is a food composition that comprises:
(a) at least about 25 mg/kg (or from about 25 to about 2,000 mg/kg, or about 40 to about 1500 mg/kg, or about 50 to about 1000 mg/kg, or about 75 to about 500 mg/kg, or about 100 to about 200 mg/kg) vitamin C, based on the dry weight of the composition;
(b) at least about 300 IU/kg (or from about 300 to about 2000 IU/kg, or about 500 to about 1400 IU/kg, or about 600 to about 1100 IU/kg, or about 700-800 IU/kg) vitamin E, based on the dry weight of the composition;
(c) less than about 25% (or less than about 20%) protein, based on the dry weight of the composition; and
(d) less than about 1.0% (or less than about 0.75%, or less than about 0.60%) phosphorus, based on the dry weight of the composition

In such an embodiment, the composition also may, for example, comprise at least one of the following:
(a) from about 0% to about 75% carbohydrate,
(b) from about 2% to about 50% fat,
(c) from about 0% to about 40% dietary fiber, and
(d) from about 0% to about 15% of one or more nutritional balancing agents.

In another embodiment, the composition comprises from about 25 to about 2000 mg/kg vitamin C, from about 300 to about 2000 IU/kg vitamin E, from about 14% to about 23% protein, and from about 0.2% to about 0.75% phosphorus.

Food compositions may be prepared in a canned or wet form using conventional food preparation processes known to skilled artisans. Typically, ground animal proteinaceous tissues are mixed with the other ingredients such as fish oils, cereal grains, balancing ingredients, special purpose additives (e.g., vitamin and mineral mixtures, inorganic salts, cellulose and beet pulp, bulking agents, and the like) and water in amounts sufficient for processing. These ingredients are mixed in a vessels suitable for heating while blending the components. Heating of the mixture is effected using any suitable manner, e.g., direct steam injection or using a vessel fitted with a heat exchanger. Following the addition of the last ingredient, the mixture is heated to a temperature of from about 50°F to about 212°F. Temperatures outside this range are acceptable but may be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. Sterilization is usually accomplished by heating to temperatures of greater than about 230°F (110°C) for an appropriate time depending on the temperature used, the composition, and similar factors. The compositions of the present invention can be added to the food compositions before, during, or after preparation.

Food compositions may be prepared in a dry form using conventional processes known to skilled artisans. Typically, dry ingredients such as animal protein, plant protein, grains, and the like are ground and mixed together. Moist or liquid ingredients, including fats, oils, animal protein, water, and the like are then added to and mixed with the dry mix. The mixture is then processed into kibbles or similar dry pieces. Kibble is often formed using an extrusion process in which the mixture of dry and wet ingredients is subjected to mechanical work at a high pressure and temperature and forced through small openings and cut off into kibble by a rotating knife. The wet kibble is then dried and optionally coated with one or more topical coatings such as flavors, fats, oils, powders, and the like. Kibble also can be made from the dough using a baking process, rather than extrusion, wherein the dough is placed into a mold before dry-heat processing. The food compositions can be in the form of a treat using an extrusion or baking process similar to those described above for dry food or a toy such as those disclosed in U.S. Patent Nos. 5,339,771 and 5,419,283. The compositions of the present invention can be added to the food compositions before, during, or after preparation.

Treats include compositions that are given to an animal to entice the animal to eat during a non-meal time, e.g., dog bones for canines. Treats may be nutritional wherein the composition comprises one or more nutrients or and may have a food-like composition. Non-nutritional treats encompass any other treats that are non-toxic. The composition or components are coated onto the treat, incorporated into the treat, or both. Treats of the present invention can be prepared by an extrusion or baking process similar to those used for dry food. Other processes also may be used to either coat the composition on the exterior of existing treat forms or inject the composition into an existing treat form.

Supplements include a feed used with another feed to improve the nutritive balance or performance of the total. Supplements include compositions that are fed undiluted as a supplement to other feeds, offered free choice with other parts of an animal's ration that are separately available, or diluted and mixed with an animal's regular feed to produce a complete feed. The AAFCO, for example, provides a discussion relating to supplements in the American Feed Control Officials, Inc. Official Publication, p. 220 (2003). Supplements may be in various forms including, for example, powders, liquids, syrups, pills, encapsulated compositions, etc.

The antioxidants of the invention may be incorporated into the composition during the processing of the formulation, such as during and/or after mixing of other components of the composition. Distribution of these components into the composition can be accomplished by conventional means.

In another aspect, the present invention provides the compositions and food compositions of the present invention further comprising one or more renal drugs. Renal drugs useful in the invention are any renal drugs known to skilled artisans to be useful for improving kidney function or combating kidney disease. Preferred drugs include lysosome-activating compounds such as those described in US Patent Number (USPN) 6,589,748, triterpene saponins such as those described in USPN 6,784,159, activin inhibitors such as those described in USPN 6,599,876 and US Patent Application Number (USPAN) 20020028762, integrin receptor inhibitors and TGF inhibitors such as those described in USPN 6,492,325, TGF activation inhibitors such as those described in USPN 6,458,767, and insulin-like growth factor (IGF) as described in USPN 5,723,441. Most Preferred drugs include Converting Enzyme (ACE) inhibitors, androgens, erythropoiten, and calcitriol. Angiotensin and endothelin are potent systemic vasoconstrictors with specific intrarenal effects that contribute to progressive renal injury. A variety of renal drugs are used to mitigate the effect of these vasoconstrictors. Angiotensin converting enzyme inhibitors (enalapril - Enacard and Vasotec and benazepril - Lotensin) have been associated with a reduction in the severity of proteinuria and slowing of progression of renal failure. The ACE inhibitor enalapril (Enacard, Vasotec) limits glomerular and systemic hypertension, proteinuria, and glomerular and tubulointerstitial lesions. Angiotensin blockers and endothelin inhibitors have beneficial effects in renal disease. Vasopeptide inhibitors are agents that inhibit both ACE and neutral endopeptidase, an enzyme involved in the breakdown of natriuretic peptides, adrenomedullin, and bradykinin. These renal drugs decrease angiotenin II production and increase accumulation of vasodilators. Renal animals with systemic hypertension respond to calcium channel blockers such as amlodipine (Norvasc). Uremic gastritis (esophagitis, gastritis, gastric ulceration and hemorrhage) is treated with H2 receptor antagonists (cimetidine - Tagamet, famotidine - Pepcid), proton pump blockers (omeprazole - Prilosec), cytoprotective agents (misoprostol - Cytotec), and antiemetic drugs that effect the emetic center (chlorpromazine - Thorazine, perchlorperazine - Compazine, metoclopramide - Reglan). Androgens or anabolic steroids (Stanozol, Winstrol-V) are used in the treatment of anemia associated with chronic renal failure. Hormone replacement therapy using recombinant human (or other species) erythropoiten (Epoetin alpha, Epogen, Procrit) is the treatment of choice for severe anemia associated with renal failure. Phosphate binders (aluminum hydroxide - Amphojel, aluminum carbonate - Basaljel) are used to control hyperphosphatemia and secondary renal hyperparathyroidism. Calcitriol (1, 25-dihydroxycholecalciferol) (Rocaltrol) and vitamin D analogues cause a calcium- independent suppression of parathyroid hormone (PTH). Administration of phosphate binders, calcitriol and related compounds has been advocated in chronic renal failure to prevent multi-system toxicity caused by PTH. Potassium depletion and hypokalemia are common in cats with chronic renal failure. Oral supplementation of potassium in the form of potassium gluconate (Tumil K, RenaKare, Kolyum) or citrate is recommended. Holistic renal drugs and compositions are also included in the present invention. Preferred holistic renal drugs include cranberry extract and mannose. Cranberry extract is purported to reduce the prevalence of urinary tract infection which is a common risk factor for long-term decline of renal function. Renal drugs include typical small molecule pharmaceuticals, small proteins, macromolecular proteins and molecules, and antibodies and further include vaccines designed to improve kidney function and/or prevent renal disease. Antibodies include polyclonal and monoclonal antibodies and immunoglobulin fragments such as Fv, Fab, Fab', F(ab')2, or other antigen-binding antibody subsequences that interact with an antigen and perform the same biological function as a native antibody. The renal drugs are administered to the animal using any method appropriate for the renal drug and in amounts known to skilled artisans to be sufficient to improve kidney function and/or treat or prevent renal disease.

In a further aspect, the description provides methods for improving kidney function. The methods comprise maintaining the animal on a diet comprising a composition comprising a kidney function improving amount of one or more antioxidants and having lower amounts of protein and phosphorus than the maximum typically recommended for a healthy animal of the same species or breed. The compositions are fed to an animal for a sufficient period to improve kidney function, e.g., until the animal shows a reduced serum nitrogen level and/or improved GFR. The compositions used in the methods contain the antioxidants and amounts of the antioxidants and the amounts of protein and phosphorus given herein for the compositions useful for improving kidney function. The method is particularly applicable to animals that are susceptible or suffering from kidney disease caused by aging, xenobiotics, or pathogens.

Generally, the methods comprise maintaining the animal on a diet comprising antioxidants, protein, and phosphorus in amounts given herein for the compositions of the present invention. In one embodiment, the method comprises administering to an animal a composition, preferably a food composition, comprising from about 25 to about 2000 mg/kg vitamin C, from about 300 to about 2000 IU/kg vitamin E, from about 14% to about 23% protein, and from about 0.2% to about 0.75% phosphorus. The compositions are administered to the animal using any suitable method, preferably by feeding the compositions to the animal.

A further method comprises administering the composition or food composition of the present invention in conjunction with one or more renal drugs. Typically, health care professionals, e.g., doctors and veterinarians, diagnose kidney disease in an animal and prescribe a renal drug (any drug useful to prevent or treat kidney disease in an animal) to treat the disease. The animal is administered the renal drug until the symptoms cease and the disease is considered cured. Generally, the renal drug is not administered after the disease is considered cured. Administration of the renal drug is resumed only if the animal has a reoccurrence of the kidney disease. In the present invention, the compositions and renal drugs are administered in conjunction to the animal during treatment. After administration of the renal drug ceases, the compositions are administered to the animal to prevent reoccurrence of the disease. In another embodiment, the compositions are administered to the animal only after use of the renal drug is discontinued to prevent disease reoccurrence.

Any suitable diagnostic method of assessing kidney function can be used to determine whether an improvement occurs. In one embodiment, kidney function is assessed by the level(s) of one or more biomarkers in a tissue or biofluid sample. A particularly useful diagnostic method herein comprises measurement of albumin content of urine. Elevated albumin levels in urine, in particular the slightly to moderately elevated levels known as microalbuminuria, are an indicator of a decline in kidney function as occurs, for example, in incipient kidney disease.

In a further aspect, the present invention provides kits suitable for administering compositions for improving kidney function to an animal. The kits comprise in separate containers in a single package or in separate containers in a virtual package, as appropriate for the kit component, at least one antioxidant and one or more of (1) one or more different antioxidants, (2) one or more ingredients suitable for consumption by an animal having a reduced amount of protein and/or phosphorus as compared to the maximum amount of protein and/or phosphorus typically recommended for a healthy animal of the same species or breed, (3) one or more renal drugs, (4) one or more renal diagnostic devices, (5) instructions for how to combine the antioxidants, ingredients, and other components to produce a composition for improving kidney function, and (6) instructions for how to use antioxidants and other components of the present invention to improve kidney function. When the kit comprises a virtual package, the kit is limited to instructions in a virtual environment in combination with one or more physical kit components. The kit contains antioxidants, ingredients, and other components in amounts sufficient to improve kidney health as described herein. Typically, antioxidants, ingredients, and the other suitable kit components are admixed just prior to consumption by an animal. The kits may contain the kit components in any of various combinations and/or mixtures. In one embodiment, the kit contains a packet containing one or more antioxidants and a container of food having a relatively reduced amount of protein and/or phosphorus for consumption by an animal. The kit may contain additional items such as a device for mixing antioxidants and ingredients or a device for containing the admixture, e.g., a food bowl. In another embodiment, antioxidants are mixed with additional nutritional supplements such as vitamins and minerals that promote good health in an animal.

In some embodiments, the kits further comprise one or more renal diagnostic devices for determining kidney function and evaluating the presence and severity of kidney disease in an animal. The renal diagnostic devices useful in the present invention include any device suitable for determining kidney function and evaluating the presence and severity of kidney disease in an animal. Preferred diagnostic methods include serum urea nitrogen (SUN), creatinine levels, urine specific gravity, and DNA damage, including urine assays for albumin such as those described in USPN 6,589,748, USPN 6,447,989 and USPAN 20050026225 and comet trail assays. Diagnostic methods are based upon known techniques including (1) blood markers such as elevated blood urea nitrogen concentration, elevated serum creatinine concentration, hyperphosphatemia, hyperkalemia or hypokalemia, metabolic acidosis and hypoalbuminemia, (2) urine markers such as impaired urine concentrating ability, proteinuria, cylinduria, renal hematuria, inappropriate urine pH, inappropriate urine glucose concentration, and cystinuria, (3) physical, imaging, and diagnostic markers such as size, shape, location, and density, (4) single nucleotide polymorphisms (SNPs) such as those disclosed in WO 2004113570 A2, (5) genetic profiles that are indicative of kidney disease, (6) proteomic profiles that are indicative of kidney disease, and (7) metabolic profiles that are indicative of kidney disease. These diagnostic methods and devices (e.g., test strips, ELISA assays, comet assays,) based upon such methods are commonly available to skilled artisans such as scientists and health care professionals and many are available to consumers, e.g., the Heska Corporation's (Fort Collins Colorado) E.R.D.-HealthScreen Urine Tests that detects small amounts of albumin in the urine ("microalbuminuria"). The kits further comprise information that the use of the compositions and methods of the present invention will improve kidney function.

In some embodiments, the kits further comprise one or more renal drugs in a separate package. In other embodiments, the kits further comprise one or more renal diagnostic devices for determining kidney function and evaluating the presence and severity of kidney disease in an animal in a separate package. The renal diagnostic devices useful in the present invention include any device suitable for determining kidney function and evaluating the presence and severity of kidney disease in an animal. Preferred diagnostic methods include serum urea nitrogen (SUN), creatinine levels, urine specific gravity, and DNA damage, including urine assays for albumin such as those described in USPN 6,589,748, USPN 6,447,989 and USPAN 20050026225 and comet trail assays. Diagnostic methods are based upon known techniques including (1) blood markers such as elevated blood urea nitrogen concentration, elevated serum creatinine concentration, hyperphosphatemia, hyperkalemia or hypokalemia, metabolic acidosis and hypoalbuminemia, (2) urine markers such as impaired urine concentrating ability, proteinuria, cylinduria, renal hematuria, inappropriate urine pH, inappropriate urine glucose concentration, and cystinuria, (3) physical, imaging, and diagnostic markers such as size, shape, location, and density, (4) single nucleotide polymorphisms (SNPs) such as those disclosed in WO 2004113570 A2, (5) genetic profiles that are indicative of kidney disease, (6) proteomic profiles that are indicative of kidney disease, and (7) metabolic profiles that are indicative of kidney disease. These diagnostic methods and devices (e.g., test strips, ELISA assays, comet assays,) based upon such methods are commonly available to skilled artisans such as scientists and health care professionals and many are available to consumers, e.g., the Heska Corporation's (Fort Collins Colorado) E.R.D.-HealthScreen Urine Tests that detects small amounts of albumin in the urine ("microalbuminuria"). The kits further comprise information that the use of the compositions and methods of the present invention will improve kidney function.

The kits of the present invention contain the compositions, composition components, food compositions, food ingredients, renal drugs, and renal diagnostic devices in any of various combinations. For example, one kit comprises a food composition comprising an admixture of one or more food ingredients and the composition in combination with a renal diagnostic device or a renal drug or both. Another kit contains the composition components in separate packages and one or more food ingredients in one or more separate packages with or without renal drugs or renal diagnostic devices in separate packages. Numerous such combinations can be constructed by the skilled artisan.

In another aspect, the description provides a means for communicating information about or instructions for one or more of (1) using compositions comprising a kidney function improving amount of one or more antioxidants and a reduced amount of protein and/or phosphorus to improve kidney function, (2) admixing and administering one or more of the compositions, composition components, food compositions, food ingredients, and renal drugs and information about or instructions for using the renal diagnostic devices of the present invention, and (3) using the kits of the present invention to improve kidney function. The means comprises a document, digital storage media, optical storage media, audio presentation, or visual display containing the information or instructions. In certain embodiments, the communicating means comprises a document, digital storage media, optical storage media, audio presentation, or visual display containing the information or instructions. Preferably, the communication is a displayed web site or a brochure, product label, package insert, advertisement, or visual display containing such information or instructions. Useful information includes one or more of (1) methods and techniques for combining and administering the compositions, composition components, food compositions, food ingredients, and renal drugs, (2) information for using the renal diagnostic devices, (3) details about the side effects, if any, caused by using the present invention in combination with other drugs, and (4) contact information for animals to use if they have a question about the invention and its use. Useful instructions include dosages, administration amounts and frequency, and administration routes. The communication means is useful for instructing an animal on the benefits of using the present invention and communicating the approved methods for administering the invention to an animal.

In a further aspect, the present invention provides for a use of a composition comprising a kidney function improving amount of one or more antioxidants and a reduced amount of protein and/or phosphorus as compared to the maximum amount of protein and/or phosphorus typically recommended for a healthy animal of the same species or breed to prepare a medicament. In another, the invention provides for the use of such composition to prepare a medicament for improving kidney function. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

The compositions, methods, and kits are useful for decreasing the morbidity and mortality for animals susceptible to or suffering from poor kidney function and for improving kidney function.

### EXAMPLES

The invention can be further illustrated by the following examples of preferred embodiments thereof, although it will be understood that these examples are included merely for purposes of illustration and are not intended to limit the scope of the invention unless otherwise specifically indicated.

### Example 1

Twenty four senior dogs were evaluated for initial GFR, weighed, and then assigned to one of three foods. Approximately two weeks prior to the study, all of the dogs were tested for microalbuminuria, had their blood pressure measured, had their GFR estimated by serum iohexol clearance, and had a DEXA analysis completed.

The three foods used in the study were a first control food, a second control food, or a dry test food for one year. Control Food 1 was modeled after a nutrient composite of a typical dry dog food in the United States. Control Food 2 was similar to Control Food 1, but phosphorus and calcium were restricted and vitamins E and C were enhanced. The Test Food was similar to Control Foods 1 and 2, but protein, calcium and phosphorus were restricted and vitamins E and C were enhanced.

On Day 1 of the study, the animal subjects had blood drawn and then were fed the appropriate food. Initial weights were recorded and daily food intake was recorded throughout the study. Animals were fed to maintain body weight throughout the year long study.

On or about Day 120 of the study, all animals were vaccinated (all killed vaccine) in a five way lepto vaccine. Approximately two weeks later, blood was drawn from the animals to ascertain vaccination response. The animals were also vaccinated again to ascertain secondary response.

After about six months of the study, GFR of the animals was estimated, a DEXA analysis was completed, blood pressure was taken and blood analysis was undertaken. This same set of analyses was performed after about one year from the beginning of the study.

The ingredients in the control foods and test food are described in Table 1.

**Table 1**

| Compositions of Control and Test Foods | | | |
|---|---|---|---|
| Ingredient | % by Weight Control Food 1 | % by Weight Control Food 2 | % by Weight Test Food |
| Corn | 61.128 | 56.659 | 65.992 |
| Chicken by-product meal | 23.68 | 14.843 | 13.70 |
| Animal Fat | 4.24 | 6.52 | 6.80 |
| Soybean mill run | 4.50 | 4.50 | 4.50 |
| Flaxseed | | 4.00 | 4.00 |
| Corn gluten meal | 1.00 | 9.60 | 1.00 |
| Egg product | 1.00 | 1.00 | 1.00 |
| Palatability enhancer | 1.00 | 1.00 | 1.00 |
| Potassium Chloride | 0.38 | 0.69 | 0.67 |
| Choline Chloride | 0.27 | 0.27 | 0.27 |
| Glyceryl Monostearate | 0.20 | 0.20 | 0.20 |
| Calcium carbonate | 0.36 | 0.10 | 0.16 |
| Salt (NaCl) | 0.66 | 0.12 | 0.16 |
| L-Tryptophan | | 0.05 | 0.05 |
| Taurine | | 0.05 | 0.05 |
| Dicalcium Phosphate | 1.45 | | 0.05 |
| L-Lysine HCl | | .05 | 0.05 |
| Micronutrient premix | 0.132 | 0.348 | 0.348 |
| Vitamins | | 0.01-0.2 | 0.01-0.2 |
| Minerals | | 0.01-0.2 | 0.01-0.2 |
| Vitamin C | | 100 ppm | 100 ppm |
| Vitamin E | 160 IU/Kg | 750 IU/Kg | 750 IU/Kg |
| Protein % | 25.3 | 25.4 | 19.4 |
| Phosphorus % | 1.0 | 0.55 | 0.55 |

The results of this experiment are shown in Table 2. As shown in Table 2, the dogs that were fed the Test Food showed an increase in GFR over the course of the one year of the test. The dogs that were fed Control Food 1, without nutrient restriction and antioxidant enhancement, exhibited a slight increase in GFR, but a significantly lower increase than the dogs that were fed the Test Food. Lastly, dogs that were fed Control Food 2, with phosphorus restriction and enhanced antioxidants, exhibited an increase in GFR over the first six months of the study, but then a significant decrease in GFR over the last six months of the study. And, as shown in Table 2, the dogs exhibited a positive correlation of concentration of creatinine and concentration of serum urea nitrogen - as the concentration of creatinine decreased, so did the concentration of serum urea nitrogen. Similarly, the dogs exhibited a positive correlation of concentration of phosphorus and concentration of serum urea nitrogen. Conversely, the dogs exhibited a negative correlation of concentration of serum urea nitrogen and GFR - as serum urea nitrogen decreased, GFR increased.

**Table 2**

| Change in Concentrations of Serum Urea Nitrogen | |
|---|---|
| | Change in Serum Urea Nitrogen |
| Change in Concentration of Creatinine | 0.68 (P<0.001) |
| Change in Concentration of Phosphorus | 0.51 (P=0.002) |
| Change in GFR | -0.40 (P=0.015) |

## Claims

1. A composition suitable for improving kidney function in an animal wherein the composition comprises at least about 10 mg/kg of one or more antioxidants, from about 14% to about 23% protein, and from about 0.2% to about 0.75% phosphorus.

2. The composition of claim 1 wherein the antioxidants are β-carotene, selenium, coenzyme Q₁₀ (ubiquinone), lutein, tocotrienols, soy isoflavones, S-adenosylmethionine, glutathione, taurine, N-acetylcysteine, vitamin E, vitamin C, α-lipoic acid and L-camitine

3. The composition of claim 1 or claim 2 wherein the antioxidants are vitamin C and vitamin E.

4. The composition of claim 3 wherein the composition comprises from about 25 to about 2000 mg/kg vitamin C and from about 300 to about 2000 IU/kg vitamin E.

5. The composition of any preceding claim wherein the animal is a canine or a feline.

6. A composition according to any preceding claim for use in improving kidney function in an animal.

7. A kit suitable for administering a composition according to any preceding claim for improving kidney function in an animal comprising in separate containers in a single package or in separate containers in a virtual package, as appropriate for the kit component, at least one antioxidant and one or more of (1) one or more different antioxidants, (2) one or more ingredients suitable for consumption by an animal having a reduced amount of protein and/or phosphorus as compared to the maximum amount of protein and/or phosphorus typically recommended for a healthy animal of the same species or breed, (3) one or more renal drugs, and (4) one or more renal diagnostic devices.

8. The kit of claim 7 comprising from about 25 to about 2000 mg/kg vitamin C in one container, from about 300 to about 2000 IU/kg vitamin E in a separate container, and a food composition comprising from about 14% to about 23% protein and from about 0.2% to about 0.75% phosphorus in a separate container.

## Patentansprüche

1. Zusammensetzung, die zur Verbesserung der Nierenfunktion bei einem Tier geeignet ist, wobei die Zusammensetzung mindestens etwa 10 mg/kg von einem oder mehreren Antioxidantien, etwa 14 % bis etwa 23 % Protein und etwa 0,2 % bis etwa 0,75 % Phosphor umfasst.

2. Zusammensetzung nach Anspruch 1, wobei die Antioxidantien β-Karotin, Selen, Coenzym Q₁₀ (Ubichinon), Lutein, Tocotrienole, Soja-Isoflavone, S-Adenosylmethionin, Glutathion, Taurin, N-Acetylcystein, Vitamin E, Vitamin C, α-Liponsäure und L-Carnitin sind.

3. Zusammensetzung nach Anspruch 1 oder Anspruch 2, wobei die Antioxidantien Vitamin C und Vitamin E sind.

4. Zusammensetzung nach Anspruch 3, wobei die Zusammensetzung etwa 25 bis etwa 2000 mg/kg Vitamin C und etwa 300 bis etwa 2000 IE/kg Vitamin E umfasst.

5. Zusammensetzung nach einem der vorhergehenden Ansprüche, wobei das Tier ein Hund oder eine Katze ist.

6. Zusammensetzung nach einem der vorhergehenden Ansprüche zur Verwendung bei der Verbesserung der Nierenfunktion bei einem Tier.

7. Kit, das geeignet ist zur Verabreichung einer Zusammensetzung gemäß einem der vorhergehenden Ansprüche zur Verbesserung der Nierenfunktion bei einem Tier, das in getrennten Behältern in einem Einzelpaket und in getrennten Behältern in einem virtuellen Paket, wie es für die Kitkomponente geeignet ist, mindestens ein Antioxidans und ein oder mehrere von (1) einem oder mehreren anderen Antioxidantien, (2) einem oder mehreren Bestandteilen, die geeignet sind zum Verzehr durch ein Tier, mit einer verringerten Menge an Protein und/oder Phosphor, verglichen mit dem Höchstgehalt an Protein und/oder Phosphor, der typischerweise für ein gesundes Tier der gleichen Spezies oder Rasse empfohlen wird, (3) einem oder mehreren Nierenarzneimitteln und (4) einem oder mehreren Nierendiagnostikgeräten umfasst.

8. Kit nach Anspruch 7, das etwa 25 bis etwa 2000 mg/kg Vitamin C in einem Behälter, etwa 300 bis etwa 2000 IE/kg Vitamin E in einem getrennten Behälter und eine Futterzusammensetzung, die etwa 14 % bis etwa 23 % Protein und etwa 0,2 % bis etwa 0,75 % Phosphor umfasst, in einem getrennten Behälter umfasst.

## Revendications

1. Composition convenant pour améliorer la fonction rénale d'un animal, laquelle composition comprend au moins environ 10 mg/kg d'un ou plusieurs antioxydants, d'environ 14 % à environ 23 % de protéine, et d'environ 0,2 % à environ 0,75 % de phosphore.

2. Composition selon la revendication 1, dans laquelle les antioxydants sont le β-carotène, le sélénium, la coenzyme Q₁₀ (ubiquinone), la lutéine, les tocotriénols, les isoflavones de soja, la S-adénosylméthionine, le glutathion, la taurine, la N-acétylcystéine, la vitamine E, la vitamine C, l'acide α-lipoïque et la L-carnitine.

3. Composition selon la revendication 1 ou 2, dans laquelle les antioxydants sont la vitamine C et la vitamine E.

4. Composition selon la revendication 3, laquelle composition comprend d'environ 25 à environ 2000 mg/kg de vitamine C et d'environ 300 à environ 2000 UI/kg de vitamine E.

5. Composition selon l'une quelconque des revendications précédentes, dans laquelle l'animal est un canidé ou un félidé.

6. Composition selon l'une quelconque des revendications précédentes, pour une utilisation dans l'amélioration de la fonction rénale d'un animal.

7. Kit convenant pour administrer une composition selon l'une quelconque des revendications précédentes, afin d'améliorer la fonction rénale d'un animal, comprenant, dans des récipients séparés dans un seul emballage ou dans des récipients séparés dans un emballage virtuel, selon ce qui est approprié pour le composant de kit, au moins un antioxydant et un ou plusieurs parmi (1) un ou plusieurs antioxydants différents, (2) un ou plusieurs ingrédients pouvant être consommés par un animal et ayant une quantité de protéine et/ou de phosphore réduite par rapport à la quantité maximale de protéine et/ou de phosphore typiquement recommandée pour un animal en bonne santé de la même espèce ou race, (3) un ou plusieurs médicaments pour les reins, et (4) un ou plusieurs dispositifs de diagnostic de la fonction rénale.

8. Kit selon la revendication 7, comprenant d'environ 25 à environ 2000 mg/kg de vitamine C dans un récipient, d'environ 300 à environ 2000 UI/kg de vitamine E dans un récipient séparé, et une composition alimentaire comprenant d'environ 14 % à environ 23 % de protéine et d'environ 0,2 % à environ 0,75 % de phosphore dans un récipient séparé.
